(19) **Europäisches Patentamt**

**European Patent Office**

**Office européen des brevets**

(11) Veröffentlichungsnummer : **0 305 357 B1**

(12) **EUROPÄISCHE PATENTSCHRIFT**

(45) Veröffentlichungstag der Patentschrift :
05.02.92 Patentblatt 92/06

(51) Int. Cl.⁵ : **A61C 1/14, A61C 1/07**

(21) Anmeldenummer : **88890215.2**

(22) Anmeldetag : **23.08.88**

(54) **Zahnärztliches Handstück.**

(30) Priorität : **24.08.87 AT 2108/87**

(43) Veröffentlichungstag der Anmeldung :
**01.03.89 Patentblatt 89/09**

(45) Bekanntmachung des Hinweises auf die
Patenterteilung :
**05.02.92 Patentblatt 92/06**

(84) Benannte Vertragsstaaten :
**CH DE FR GB IT LI**

(56) Entgegenhaltungen :
**WO-A-84/01099
DE-C- 969 644
FR-A- 1 541 927
US-A- 4 571 183**

(73) Patentinhaber : **DENTALWERK BÜRMOOS
GESELLSCHAFT M.B.H.
Ignaz-Glaser-Strasse 53
A-5111 Bürmoos (AT)**

(72) Erfinder : **Apap, Marc François Charles
33, rue Carvès
F-92120 Montrouge (FR)**
Erfinder : **Thorin, Cedric Louis Marie Christian
Rue de Seine
F-75006 Paris (FR)**
Erfinder : **Rosenstatter, Otto
Matzing 105
A-5164 Seeham (AT)**
Erfinder : **Malata, jun., Peter, Dipl.-Ing.
Ignaz Glaser-Strasse 55
A-5111 Bürmoos (AT)**

(74) Vertreter : **Barger, Erich et al
Patentanwälte Dipl.-Ing. Erich Barger
Dipl.-Ing. Hermann Krick Biberstrasse 15
A-1010 Wien (AT)**

**Beschreibung**

Die Erfindung betrifft ein zahnärztliches Handstück für ein endodontisches Feilwerkzeug, welches im Handstück, sowohl gegen rotierende als auch gegen axiale Bewegung gesichert, eingespannt wird. Dem Werkzeug wird eine Vibrationsbewegung aufgeprägt.

Solche Wurzelkanalfeilen sind aus der US-PS 4571183 und der DE-OS-3337367 bekannt. Die Feile gemäß der US-PS führt dabei eine kombinierte Vibration quer zur und längs der Feilenachse aus, auf die besonderer Wert gelegt wird. Die Schwingungen bei der Vorrichtung gemäß der DE-OS werden gemäß US-PS 4330278 oder FR-PS 2505172 erzeugt und sind völlig undefiniert, doch kommt es aufgrund der Geometrie des Handstückes immer auch zu merklichen Längsschwingungen des Werkzeuges.

Neben solchen Feilwerkzeugen sind Geräte bekannt, die mittels oszillierender Drehbewegung arbeiten, der gegebenenfalls eine axiale, pendelnde Hubbewegung überlagert sein kann. Die Amplitude dieser Bewegung ist um mehrere Größenordnungen größer als bei den oben erwähnten Feilen. Durch die Eigensteifigkeit des Feilwerkzeuges werden in gekrümmten Wurzelkanälen nicht alle Wandbereiche erreicht und somit die Reinigung nur mangelhaft durchgeführt. Beim Festklemmen des dünnen Feilwerkzeuges im Kanal kann dieses durch die oszillierende Drehbewegung abgedreht werden. Bei Geräten mit pendelnder Hubbewegung besteht darüberhinaus die Gefahr, daß der Boden des Wurzelkanals durchstoßen wird und der Zahn verloren geht.

Bei Feilwerkzeugen der eingangs erwähnten Art können diese Nachteile nur in geringem Maß auftreten. Bisher wurden solche Werkzeuge mit Frequenzen zwischen 3 kHz und 20 kHz, also hochfrequent, erregt.

Dabei tritt als Nachteil auf, daß das Feilwerkzeug leicht überlastet wird und bricht. Auch ist trotz der hohen Frequenz beim Feilwerkzeug, wenn es in den Wurzelkanal eingebracht ist, eine Abnahme der Amplitude der Schwingungen mit zunehmendem Abstand vom Werkzeughalter zu beobachten, d.h., daß im unteren Bereich des Wurzelkanals nur ein ungenügender Reinigungseffekt erzielt wird. Darüberhinaus besteht die Gefahr, daß das Werkzeug sich ins Dentin eingräbt und so seinen eigenen, falschen Kanal bohrt.

Die Nervenkanäle im menschlichen Zahn haben unterschiedlichste anatomische Formen : Kreisrunde bis stark ovale Querschnitte, gerade bis stark gekrümmte Längsformen, unterschiedliche Anzahl von Ausgängen im Apexbereich, unterschiedliche Längen der Kanäle und starke Querschnittsverjüngungen im Apexbereich sind anzutreffen.

Die Erfindung hat es sich zur Aufgabe gemacht, ein zahnärztliches Handstück für ein endodontisches Feilwerkzeug, bei dem der Schaft des Werkzeuges sowohl gegen rotierende als auch gegen axiale Bewegung gesichert gehalten ist, wobei eine Erregereinrichtung im Handstück vorgesehen ist, welche das Werkzeug in eine Bewegung versetzt, die eine Komponente quer zur Werkzeugachse aufweist, zu schaffen, welches die oben genannten Nachteile nicht aufweist und für alle Wurzelkanalformen geeignet ist.

Erfindungsgemäß wird dabei vorgesehen, daß das Werkzeug an seinem hinteren Schaftende mit geringem Spiel gehalten ist und mit seinem vorderen Schaftende durch eine zylindrische Bohrung mit im wesentlichen gleichdick- oder polygonförmigen Querschnitt hindurchragt, wobei die Bohrung in einem im Handstückkopf drehbar gelagerten Bauteil als Erregereinrichtung angeordnet ist, der in bekannter Weise, z.B. mittels eines Winkeltriebes, antreibbar ist.

Die Amplitude der so vermittelten Vibration nimmt auch im Wurzelkanal zum Werkzeugende hin kaum ab und ermöglicht dadurch eine bessere Reinigung als bisher möglich. Darüberhinaus ist die Gefahr des Feilenbruches und des Bohrens eines eigenen Kanals ins Dentin ausserordentlich reduziert.

Bevorzugt läuft der Erregerbauteil im Betrieb mit etwa 20000 U/min um. Damit ergibt sich eine Pendelfrequenz von etwa 1 kHz, die sich gegenüber den bekannten Geräten mit hochfrequentem Antrieb als wesentlich sicherer herausgestellt hat.

Durch die erfindungsgemäße Vorrichtung erreicht man überraschenderweise, daß eine nahe am Feilwerkzeug austretende Kühl- und Reinigungsflüssigkeit, deren Strahl auf das Feilwerkzeug gerichtet ist, dieses umhüllt und bis zum Ende des Feilwerkzeuges an diesem abläuft und die durch die Erweiterung des Wurzelkanals entstandenen Rückstände abtransportiert.

Da bei der medizinischen Applikation, wie bereits gesagt, unbedingt verhindert werden muß, daß die Spitze des Feilwerkzeuges den Apex durchstößt, aber andererseits die Höhe des Apex vom Feilwerkzeug sicher erreicht werden muß, damit der gesamte Kanal gereinigt, erweitert und in der Folge vollständig gefüllt werden kann, ist in einer Ausgestaltung der Erfindung vorgesehen, am Winkelstückkopf einen verstellbaren Tiefenanschlag anzubringen, der die verfügbare Arbeitslänge des Feilwerkzeuges, vorzugsweise um bis zu 9 mm reduzieren kann.

Da die meisten handelsüblichen Feilwerkzeuge Gesamtarbeitlängen von 21, 25 und 28 mm aufweisen, können bei einem Tiefenanschlag, der zwischen 0 und 9 mm verstellbar ist, Nervenkanäle mit Tiefen von 12 bis 28 mm behandelt werden. Dies ist für die überwiegende Anzahl der vorkommenden Behandlungen ausreichend. Es kann jedoch selbstverständlich ein zusätzlicher Tiefenanschlag für Spezialfälle vorgesehen werden.

Eine Schwierigkeit beim Bearbeiten von Wurzelkanälen liegt im häufigen Werkzeugwechsel, wobei es notwendig ist, das kleine und schwer zu handhabende Feilwerkzeug in der richtigen Winkelposition in das Halterungssystem des Winkelhandstückes einzusetzen.

In einer Ausgestaltung der Erfindung ist aus diesem Grund vorgesehen, am Schaft der Feile eine Vielzahl von kalottenförmigen Vertiefungen, die regelmäßig um den Umfang des Schaftes verteilt sind, vorzusehen, die mit Spannkugeln eines Spannmechanismus zusammenwirken und die durch das Schnellspannsystem radial verschieblich sind. Es werden bevorzugt acht kalottenförmigen Vertiefungen und zwei Spannkugeln vorgesehen.

Im betriebsbereiten Zustand muß zwischen den Vertiefungen und den Spannkugeln ein minimales Spiel bleiben, welches das beschriebene Auspendeln des Werkzeugschaftes ermöglicht.

Die Erfindung wird an Hand der Zeichnungen, die ein Ausfürhungsbeispiel zeigen, näher erläutert. Dabei zeigt

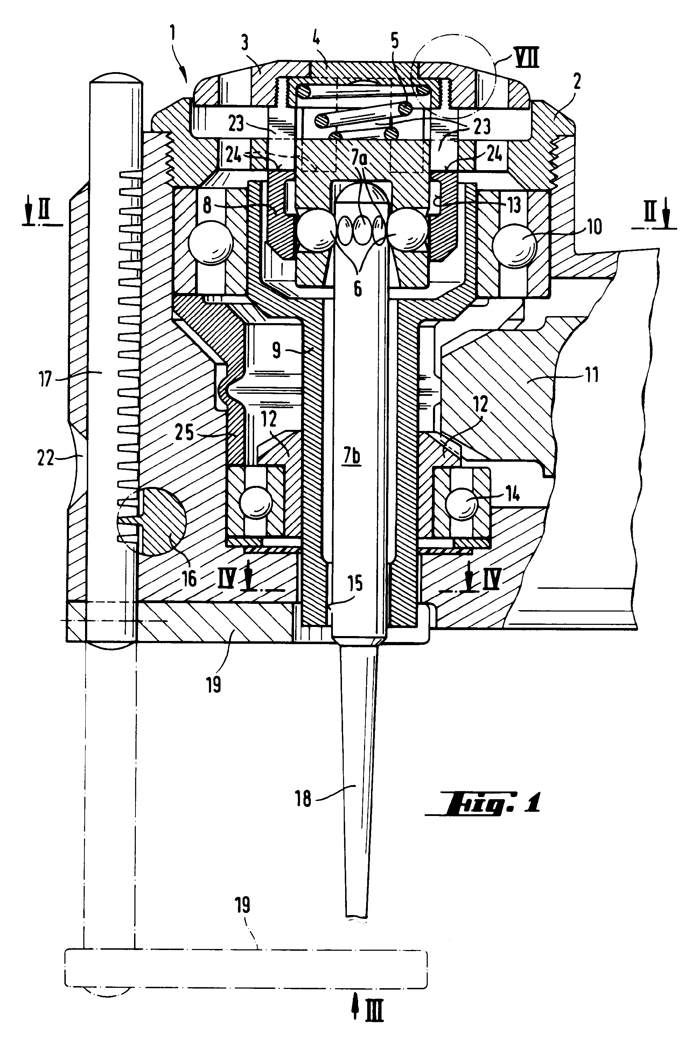

Fig. 1 einen Längsschnitt durch den Feilwinkelkopf mit eingesetztem Werkzeug ;

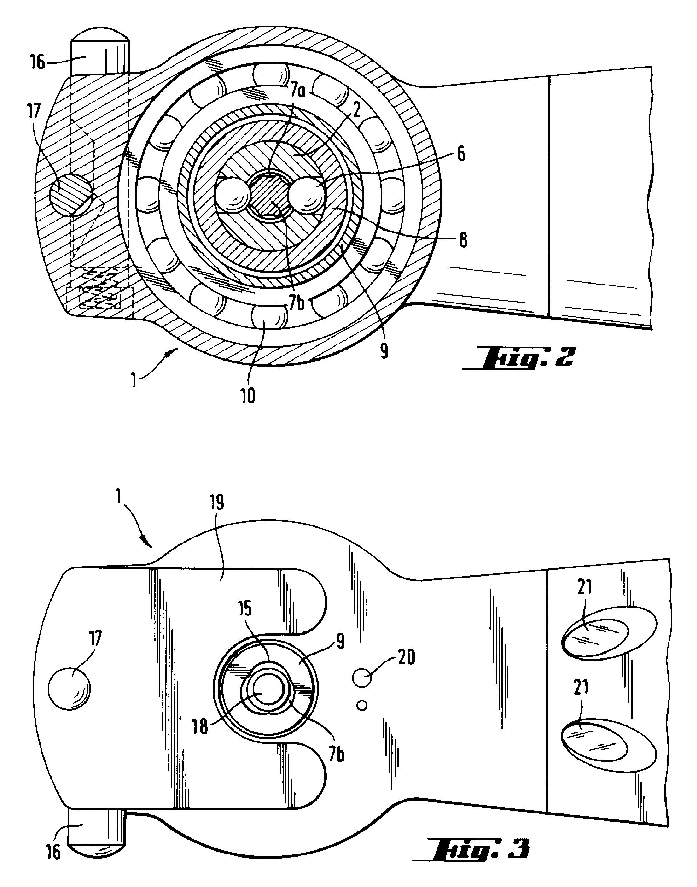

Fig. 2 einen Schnitt entlang II-II in Fig. 1 ;

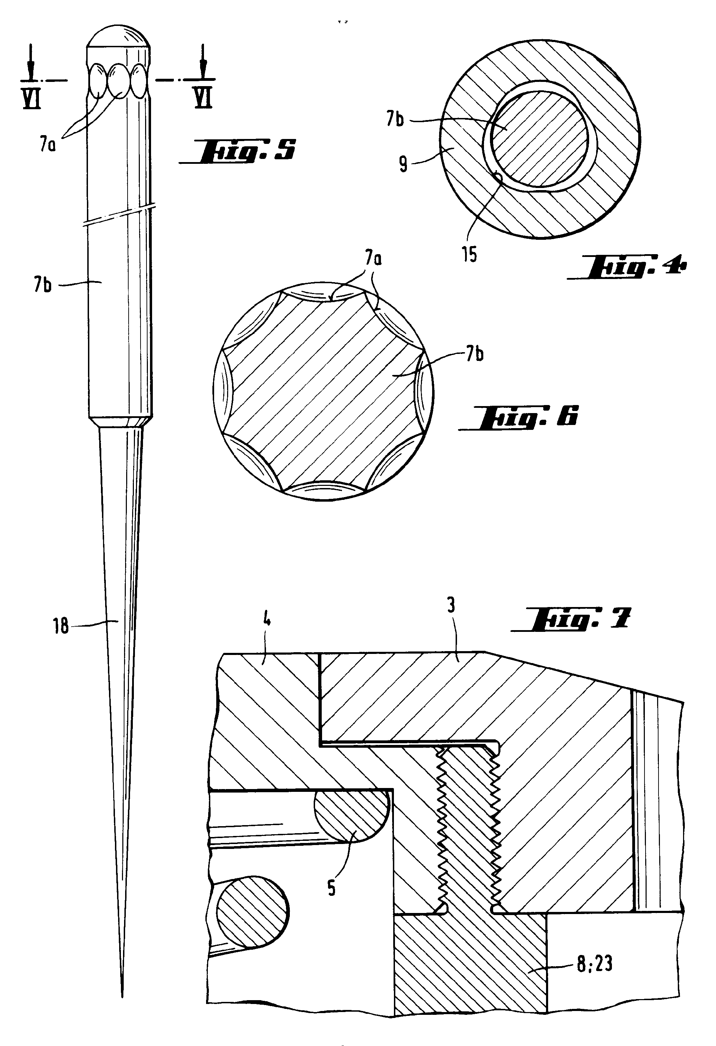

Fig. 3 eine Ansicht des Behandlungskopfes in Richtung des Pfeiles III in Fig. 1 ;

Fig. 4 einen Schnitt entlang IV-IV in Fig. 1 ; und

Fig. 5 ein Feilwerkzeug mit Schaft und Nadel,

Fig. 6 einen Schnitt durch ein Feilwerkzeug entlang der Linie VI-VI in Fig. 5 und

Fig. 7 eine Darstellung des Details VII in Fig 1 in vergrößertem Maßstab.

Im Kopf 1 eines zahnärztlichen Winkelhandstückes ist ein festsitzender Werkzeughalter 2 eingeschraubt. Eine zylindrische Spannhülse 8 ragt mit vier Stegen 23 durch den Werkzeughalter 2 und wird mit dem zweiteiligen Doppeldruckdeckel 3, 4 verschraubt. Eine Kegeldruckfeder 5 drängt den Spannmechanismus 8, 4, 3 nach oben, bis ausgesparte Planflächen 24 der Spannhülse 8 am Werkzeughalter 2 anschlagen. In dieser Spannposition werden zwei Spannkugeln 6 durch die Hülse 8 radial soweit nach innen in Kalotten 7a des Werkzeugschaftes 7b gedrückt, daß gerade noch ein minimales Spiel bleibt, wodurch die Beweglichkeit des Werkzeugschaftes sichergestellt ist.

Wenn der Deckel 3, 4 nach unten gedrückt wird, wird eine Ringnut 13 auf der Innenseite der Spannhülse 8 auf die Höhe der Spannkugeln 6 geschoben. Diese können somit radial nach außen gelangen und geben den Schaft 7b frei.

Durch die Anordnung von acht Kalotten 7a am Schaft 7b kann die Feilnadel in jeder Winkellage gespannt werden.

Dieses mit dem Handstück fest verbundene Spannsystem wird von einer rotierenden Antriebshülse 9 umgeben, welche am unteren Ende eine zylindrische Ausnehmung aufweist, deren Querschnitt die Form eines Gleichdicks 15 besitzt. Zwischen diesem Gleichdick und dem Werkzeugschaft 7b besteht minimales Spiel, welches jedoch beim Rotieren der Antriebshülse 9 einen Kontakt zwischen Schaft und Hülse nicht verhindert.

Die dem Werkzeug aufgeprägte Vibrationsbewegung ist pendelnd und aleatorisch (unregelmäßigzufallsbedingt) ; eine Bewegung des Werkzeuges in Richtung der Feilenachse erfolgt nicht.

Die Antriebshülse 9 ist in Kugellagern 10, 14 gelagert, die durch den Werkzeughalter 2 und einen Abstandshalter 25 axial fixiert sind. Der Antrieb der Hülse 9 erfolgt über einen aus Zahnrädern 11, 12 bestehenden Winkeltrieb.

Die maximal wirksame Arbeitslänge des Feilwerkzeuges kann durch einen Anschlag 19 begrenzt werden. Der Anschlag 19 wird mittels einer Stange 17 im Gehäuse 1 geführt, die durch einen Querriegel 16 in der jeweils gewünschten Position fixiert ist. Die Stange 17 ist mit einem Maßstab versehen, der es erlaubt, die jeweilige Tiefenlage des Anschlages an einer Aussparung 22 des Kopfes abzulesen. Bevorzugt sind Verstellintervalle von 0,5 mm vorgesehen. Der Anschlag 19 ist vorteilhafterweise so ausgestaltet, daß er den Zutritt der Reinigungs- und Kühlflüssigkeit von der Austrittsstelle 20 zum Werkzeug 18 nicht behindert und daß die Spitze des Feilwerkzeuges 18 durch zwei Lichtleiterenden 21 ausreichend erhellt wird.

Die Erfindung ist nicht auf das dargestellte Ausführungsbeispiel beschränkt. So kann ein anderes Gleichdick gewählt werden, der Spannmechanismus kann anders sein und der Tiefenanschlag braucht nicht vorgesehen zu werden oder kann eine andere Form erhalten. Auch ist es möglich, den Werkzeugschaft in Form eines Gleichdicks auszubilden und die Führung kreisrund zu gestalten.

Wesentlich an der Erfindung ist die Lagerung des oberen Endes des Werkzeugschaftes mit Spiel und die Erregung des unteren Endes des Werkzeugschaftes mittels eines mit Spiel umlaufenden Gleichdicks. Die Drehzahl des Gleichdicks liegt, wenn ein dreiseitiges Gleichdick verwendet wird, bevorzugt bei etwa 20.000 U/min, was zu Werkzeugfrequenzen um etwa 1 kHz führt.

Die erwähnten Toleranzen und Spiele können vom Fachmann in Kenntnis der Erfindung leicht für die jeweiligen Feilen und Handstücke ermittelt werden. Ebenso können die günstigsten Drehzahlen für andersförmige Gleichdicke in Kenntnis der Erfindung vom Fachmann, gegebenenfalls durch einfache Versuche, leicht bestimmt werden.

## Patentansprüche

1. Zahnärztliches Handstück mit einem endodontischen Feilwerkzeug (18), bei dem der Schaft des Werkzeuges sowohl gegen rotierende als auch gegen axiale Bewegung gesichert gehalten ist, wobei eine Erregereinrichtung im Handstück vorgesehen ist, welche das Werkzeug in eine Bewegung versetzt, die eine Komponente quer zur Werkzeugachse aufweist, dadurch gekennzeichnet, daß das Werkzeug (18) an seinem hinteren Schaftende durch zumindest zwei Spannkugeln (6) des Werkzeughalters (2), die in zumindest einer Kalottenförmige Ausnehmung (7a) des Werkzeugschaftes eingreifen, mit geringem Spiel gehalten ist und mit seinem vorderen Schaftende (7b) durch eine zylindrische Bohrung mit im wesentlichen gleichdickoder polygonförmigen Querschnitt hindurchragt, wobei diese Bohrung in einem im Handstückkopf drehbar gelagerten Bauteil (9) als Erregereinrichtung angeordnet ist, der in bekannter Weise, z.B. mittels eines Winkeltriebes (11, 12), antreibbar ist.

2. Handstück nach Anspruch 1, dadurch gekennzeichnet, daß die zumindest eine Ausnehmung (7a) Kalottenform aufweist, wobei jede Spannkugel (6) mittels einer unter der Wirkung einer Feder (5) stehenden Spannhülse (8) in eine dieser Ausnehmungen (7a) eingreift.

3. Handstück nach Anspruch 2, dadurch gekennzeichnet, daß zwei Spannkugeln (6) und acht Kalotten (7a) vorgesehen sind.

4. Handstück nach Anspruch 1, dadurch gekennzeichnet, daß, wie an sich bekannt, ein Anschlag (19) zur Begrenzung der Eindringtiefe des Werkzeuges in den Wurzelkanal am Handstück angebracht ist, mit einem Verstellintervall von vorzugsweise 0 bis 9 mm.

5. Handstück nach Anspruch 4, dadurch gekennzeichnet, daß der Anschlag (19) mittels einer gezahnten Stange (17) im Gehäuse (1) geführt und durch einen federbelasteten und mit der Verzahnung der Stange kämmenden, von Hand zu betätigenden Querriegel (16) in der jeweils gewünschten Stellung feststellbar ist, wobei die Stange einen Maßstab aufweist, der durch eine Aussparung (22) des Handstückkopfes sichtbar ist, wobei ferner bevorzugt die Zahnteilung 0,5 mm beträgt.

6. Handstück nach Anspruch 5 mit Kühl- und Behandlungsflüssigkeitszufuhr und Beleuchtungsvorrichtung, dadurch gekennzeichnet, daß der Auslaß (20) für die Flüssigkeit schräg auf das Werkzeug (18) gerichtet ist und der Anschlag (19) weder im Bereich des Flüssigkeitsstrahles noch des Lichtstrahles angeordnet und vorzugsweise hufeisenförmig ausgebildet ist.

7. Handstück nach einem der Ansprüche 1 bis 6, dadurch gekennzeichnet, daß die Begrenzung des Querschnittes der zylindrischen Bohrung drei untereinander gleiche Kreisbogenabschnitte aufweist.

8. Handstück nach Anspruch 7, dadurch gekennzeichnet, daß der die zylindrische gleichdickförmige Bohrung aufweisende Bauteil (9) im Betrieb mit etwa 20.000 U/min rotiert.

**Claims**

1. Dental handpiece with an endodontic filing tool (18), on which the shank of the tool is held secured against both rotational and axial movement, an exciting device being provided in the handpiece, which sets the tool into motion and which has a component at right angles to the tool axis, characterized in that the tool (18) is held with little play at its rear shank end by at least two tension balls (6) of the tool holder (2) which engage in at least one cup-shaped recess (7a) of the tool shank and with its front shank end (7b) projects through a cylindrical bore with substantially a cross section of equal thickness or polygon shape, this bore being disposed as an exciting device in a component (9) which is rotatably mounted in the handpiece head and which can be driven in known manner, e.g. by means of an angular drive (11, 12).

2. Handpiece according to claim 1, characterized in that the at least one recess (7a) has a cup shape with each tension ball (6) engaging in one of these recesses (7a) by means of a clamping sleeve which is under the action of a spring (5).

3. Handpiece according to claim 2, characterized in that two tension balls (6) and eight cups (7a) are provided.

4. Handpiece according to claim 1, characterized in that, as is known in itself, a stop (19) is fitted to the handpiece to limit the depth of penetration of the tool into the root canal with an adjustment interval of preferably 0 to 9 mm.

5. Handpiece according to claim 4, characterized in that the stop (19) is guided by means of a rack (17) in the housing (1) and can be fixed in any desired position by means of a manually operated, spring-loaded traverse lock (16), which meshes with the toothing of the rack, the rack having a scale which is visible through a cutout (22) of the handpiece head, the preferred tooth pitch being 0.5 mm.

6. Handpiece according to claim 5 with cooling and treatment liquid supply and lighting unit, characterized in that the outlet (20) for the liquid is directed obliquely on to the tool (18) and the stop (19) is neither disposed in the area of the liquid jet nor of the light beam and is constructed preferably in the form of a horseshoe.

7. Handpiece according to one of claims 1 to 6, characterized in that the limit of the cross section of the cylindrical bore has three circular arc sections which are equal amongst themselves.

8. Handpiece according to claim 7, characterized in that the component (9) provided with the cylindrical bore of equal thickness shape rotates in operation at a speed of 20,000 r.p.m.

## Revendications

1. Porte-outil dentaire avec un outil endodontique pour limer (18), dans lequel la queue de l'outil est maintenue de façon sûre aussi bien à l'égard d'un mouvement de rotation qu'à l'égard d'un mouvement axial, porte-outil dans lequel est prévu un dispositif d'excitation qui déplace l'outil selon un mouvement qui présente une composante perpendiculaire à l'axe de l'outil, porte-outil caractérisé par le fait que l'outil (18) est tenu, avec un faible jeu, à l'extrémité arrière de sa queue, par au moins deux billes de bridage (6) du support d'outil (2) qui viennent en prise dans au moins un évidement, en forme de calotte (7a) de la queue de l'outil et que, par l'extrémité avant (7b) de sa queue, l'outil traverse un alésage cylindrique dont la section a sensiblement la forme d'une enveloppe extérieure de trois cercles sécants symétriques ou d'un polygone, étant précisé que cet alésage est disposé, en tant que dispositif d'excitation, dans un élément (9) qui est porté, avec liberté de rotation, dans la tête du porte-outil et qui peut être entraîné de façon connue, par exemple au moyen d'un mécanisme d'entraînement d'équerre (11, 12).

2. Porte-outil selon la revendication 1, caractérisé par le fait que l'évidement (7a), dont il y a au moins un, présente la forme d'une calotte, étant précisé que chaque bille de bridage (6) vient en prise dans l'un de ses évidements (7a) au moyen d'une douille de bridage (8) qui se tient sous l'action d'un ressort (5).

3. Porte-outil selon la revendication 2, caractérisé par le fait qu'il est prévu deux billes de bridage (6) et huit calottes (7a).

4. Porte-outil selon la revendication 1, caractérisé par le fait, que comme connu en soi, une butée (19), pour limiter la profondeur de pénétration de l'outil dans le canal radiculaire, est prévue sur le porte-outil avec un intervalle de réglage, de préférence, de 0 à 9 mm.

5. Porte-outil selon la revendication 4, caractérisé par le fait que la butée (19) est guidée dans le boîtier (1) au moyen d'une crémaillère (17) et peut être bloqué dans la position chaque fois désirée au moyen d'un verrou transversal (16), contraint par un ressort, engrenant avec la denture de la crémaillère, à manoeuvrer à la main, étant précisé que la crémaillère présente une échelle que l'on peut voir par un évidement (22) de la tête du porte-outil, étant précisé en outre que de préférence la division de la denture vaut 0,5 mm.

6. Porte-outil selon la revendication 5 avec amenée de liquide de refroidissement et de traitement et avec dispositif d'éclairage, porte-outil caractérisé par le fait que la sortie (20) du liquide est orientée obliquement par rapport à l'outil (18) et que la butée (19) n'est disposée ni sur le trajet du jet du liquide ni sur le trajet du faisceau lumineux et qu'elle a de préférence la forme d'un fer à cheval.

7. Porte-outil selon l'une des revendications 1 à 6, caractérisé par le fait que l'enveloppe de la section de l'alésage cylindrique présente trois portions d'arc de cercle identiques entre elles.

8. Porte-outil selon la revendication 7, caractérisé par le fait que l'élément (9) qui présente l'alésage cylindrique en forme d'enveloppe extérieure de 3 cercles sécants symétriques tourne, en service, à une vitesse de rotation d'environ 20.000 tours/minute.

**Fig. 1**

*Fig. 2*

*Fig. 3*

Fig. 5

Fig. 4

Fig. 6

Fig. 7